# FASCICULE DE BREVET EUROPEEN

(11) **EP 2 633 025 B1**
(45) Date de publication et mention de la délivrance du brevet: **08.11.2017**
(21) Numéro de dépôt: 11787920.5
(22) Date de dépôt: 19.10.2011
(51) Int. Cl.: C12M 1/26

(54) **DISPOSITIF PORTABLE DE COLLECTE DE PARTICULES ET DE MICROORGANISMES**
TRAGBARE VORRICHTUNG ZUM SAMMELN VON PARTIKELN UND MIKROORGANISMEN
PORTABLE DEVICE FOR COLLECTING PARTICLES AND MICROORGANISMS

(30) Priorité: 27.10.2010 FR 1058870
(43) Date de publication de la demande: 04.09.2013
(73) Titulaire: Bertin Technologies, 78180 Montigny-le-Bretonneux (FR)
(72) Inventeur: VERDIER, Amandine, F-92240 Malakoff (FR); TROUCHET, Daniel, F-75015 Paris (FR); CHARPENTIER, Julien, F-92800 Puteaux (FR); VALLAYER, Bruno, 13320 BOUC-BEL-AIR (FR)
(74) Mandataire: Ramey, Daniel
(86) Numéro de dépôt international: PCT/FR2011/052445
(87) Numéro de publication internationale: WO 2012/056151

(56) Documents cités:
- EP-A1- 2 100 637
- FR-A1- 2 905 379
- US-A- 4 350 507
- US-A- 5 861 053

## Description

La présente invention concerne un dispositif portable de collecte de particules et de microorganismes présents dans l'air ambiant, à des fins d'identification et de dénombrement de ces particules et microorganismes.

Cette identification et ce dénombrement sont importants dans de nombreux domaines tels que l'industrie pharmaceutique, l'industrie agro-alimentaire, le milieu médical, les services d'hygiène, les services vétérinaires, la surveillance de sites, etc, les dimensions des particules et microorganismes à collecter étant comprises entre 0,5 µm et plusieurs dizaines de microns.

On connaît, par le document FR-A-2 855 831 ou par le document FR 2 905 379 au nom de la Demanderesse, des dispositifs de ce type qui comprennent une enceinte amovible de centrifugation contenant un liquide de collecte, associée à des moyens d'aspiration d'air. L'enceinte comprend une entrée et une sortie d'air et forme un récipient de transport d'un échantillon liquide contenant les particules et microorganismes collectés.

Il s'agit de dispositifs autonomes qui, bien que portatifs, présentent un encombrement relativement important. En fonctionnement, le débit d'air passant dans l'enceinte est relativement important, c'est-à-dire supérieur à 100 litres par minute. Le débit d'air est fonction des applications et est en général déterminé pour effectuer des prélèvements fiables et représentatifs en quelques minutes.

Les personnes devant évoluer dans une atmosphère contaminée ou qui sont confrontées à des risques nucléaires, biologiques et/ou chimiques, sont tenues de porter des appareils de protection respiratoire qui peuvent être équipés de moyens filtrants, purifiant l'air ambiant par filtration.

Ces appareils peuvent être à ventilation libre, si l'air ne traverse les filtres que du seul fait des échanges respiratoires de l'utilisateur, ou à ventilation assistée, si l'air ambiant est aspiré au travers des filtres à l'aide de moyens motorisés.

Les appareils respiratoires connus ne permettent pas d'analyser l'atmosphère dans laquelle les utilisateurs ont évolué. Lorsque l'on veut effectuer une telle analyse, l'utilisateur doit emmener avec lui un dispositif de collecte dédié, du type de ceux décrits dans les documents FR-A-2 855 831 et FR 2 905 379 précités, et le faire fonctionner sur place, ce qui n'est pas possible dans certains contextes.

L'invention a notamment pour but d'apporter une solution simple, efficace et économique à ce problème.

A cet effet, elle propose un appareil individuel motorisé de protection respiratoire équipant un gilet destiné à être porté par une personne, comportant un masque, destiné à être porté par un utilisateur, et des moyens motorisés d'alimentation en air, par exemple à turbine, permettant d'aspirer de l'air extérieur au travers d'au moins un filtre destiné à retenir les particules ou les microorganismes présents dans l'air extérieur, caractérisé en ce qu'il comporte un dispositif portable de collecte de particules et de microorganismes présents dans l'air ambiant, monté sur le filtre, en amont de celui-ci dans le sens d'aspiration de l'air extérieur, le dispositif de collecte comportant une enceinte cyclonique de centrifugation d'air de forme conique ou tronconique, des moyens d'entrée d'air extérieur dans l'enceinte, des moyens de sortie d'air de l'enceinte, et des moyens de raccordement des moyens de sortie d'air de l'enceinte à des moyens d'entrée d'air de l'appareil individuel motorisé de protection respiratoire, les moyens de raccordement de la sortie d'air de l'enceinte comprennent des moyens de branchement sur au moins un filtre d'entrée de l'appareil de protection respiratoire, les moyens de branchement comportent au moins un embout de montage en forme de cloche, venant s'emboîter directement sur le filtre d'entrée de l'appareil.

Le dispositif de collecte est associé à un appareil de protection respiratoire d'une façon permettant une collecte de particules et de microorganismes de façon continue, tout au long de la mission de l'utilisateur, et pendant toute la durée de fonctionnement de l'appareil de protection respiratoire.

Le dispositif de collecte peut être monté et démonté très facilement sur le filtre.

Selon une autre caractéristique de l'invention, la surface interne de l'enceinte cyclonique est constituée ou est recouverte d'un matériau électrostatique ou d'un matériau à base de nanofibres, destiné à collecter les particules ou les microorganismes.

Ce matériau peut par exemple être un « électret », qui est un matériau diélectrique présentant un état de polarisation quasi permanent.

De façon classique, les moyens d'entrée d'air extérieur débouchent tangentiellement dans la partie supérieure de l'enceinte et les moyens de sortie d'air débouchent axialement dans la partie supérieure de l'enceinte.

Dans un mode de réalisation préféré de l'invention, la partie supérieure de l'enceinte a un diamètre compris entre 10 et 50 mm, et la hauteur ou la dimension axiale de l'enceinte est comprise entre 10 et 100 mm.

L'invention concerne de plus un appareil individuel motorisé de protection respiratoire comportant un masque, destiné à être porté par un utilisateur, et des moyens motorisés d'alimentation en air, par exemple à turbine, permettant d'aspirer de l'air extérieur au travers d'au moins un filtre destiné à retenir les particules ou les microorganismes présents dans l'air extérieur, caractérisé en ce qu'un dispositif de collecte du type précité est monté sur le filtre, en amont de celui-ci dans le sens d'aspiration de l'air extérieur.

Avantageusement, les moyens d'alimentation en air, le filtre et le dispositif de collecte sont dimensionnés pour fournir à l'utilisateur un débit d'air continu inférieur à 100 litres par minute, correspondant à ses besoins respiratoires.

De préférence, l'appareil est destiné à être porté et utilisé pendant des périodes de temps continues pouvant atteindre 8 à 10 heures environ, la collecte des particules et des microorganismes de l'air extérieur étant faite en continu et à sec dans l'enceinte cyclonique du dispositif précité pendant toute la durée d'utilisation de l'appareil de protection respiratoire.

En variante, le dispositif de collecte selon l'invention peut être raccordé à l'entrée d'air d'une turbine d'aspiration à moteur alimenté par des batteries électriques, l'ensemble étant destiné à être porté par une personne et à fonctionner en continu pendant une durée de plusieurs heures, par exemple d'une demi-journée environ.

L'invention concerne également un procédé de collecte et de dosage des particules et des microorganismes présents dans l'air ambiant, caractérisé en ce qu'il consiste, après utilisation de l'appareil de protection respiratoire précité, à démonter l'enceinte cyclonique, à rincer sa surface interne avec un liquide approprié afin de récupérer les particules et/ou les microorganismes collectés sur cette surface, et à analyser et/ou doser le contenu de l'échantillon liquide ainsi obtenu.

Ce procédé peut également consister à soumettre l'enceinte à des ultrasons, après ou lors de son rinçage, de façon à décoller les particules et/ou les microorganismes de sa surface interne.

L'invention sera mieux comprise et d'autres détails, caractéristiques et avantages de l'invention apparaîtront à la lecture de la description suivante faite à titre d'exemple non limitatif en référence aux dessins annexés dans lesquels :
- la figure 1 est une vue schématique représentant une personne portant un appareil individuel motorisé de protection respiratoire de l'art antérieur ;
- la figure 2 est une vue en perspective d'un appareil de protection respiratoire selon l'invention,
- la figure 3 est une vue en perspective d'un dispositif de collecte selon l'invention,
- la figure 4 est une vue en coupe axiale d'une enceinte cyclonique de centrifugation d'air.
- la figure 5 est une vue en perspective d'une variante de réalisation de l'invention.

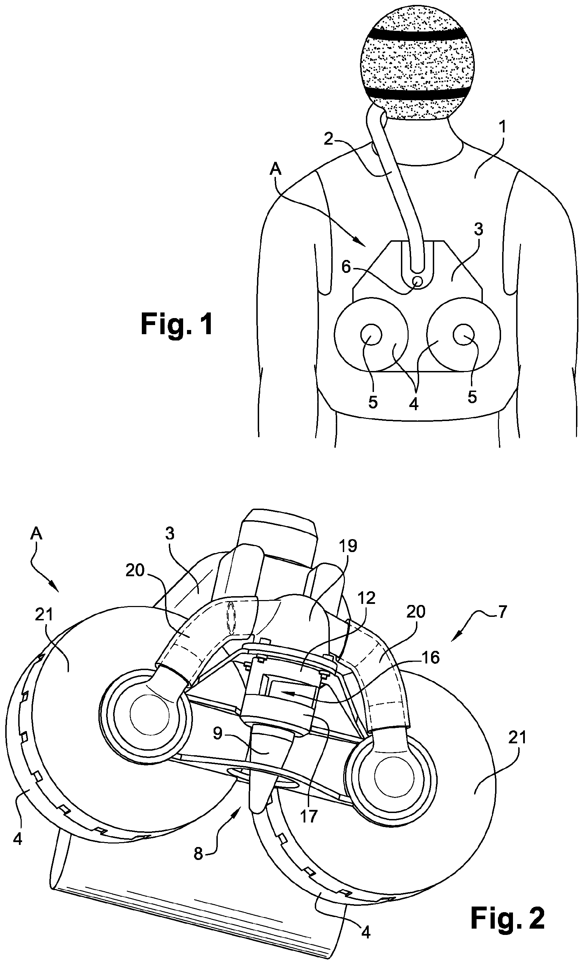

La figure 1 représente une personne portant un gilet 1 dont le dos est équipé d'un appareil individuel motorisé de protection respiratoire A comportant un masque (non visible) raccordé par un conduit 2 à des moyens motorisés d'alimentation en air 3, par exemple à turbine, permettant d'aspirer de l'air extérieur au travers de deux filtres 4 destinés à retenir les particules ou les microorganismes présents dans l'atmosphère environnante.

Chaque filtre 4 est de forme générale cylindrique et comporte une entrée d'air centrale 5 située sur sa face arrière.

L'appareil A est équipé de moyens de commande 6 permettant de démarrer ou d'arrêter les moyens motorisés 3 d'alimentation en air, et de régler le débit d'air alimentant le masque.

Comme cela est représenté à la figure 2, l'invention propose d'équiper un tel appareil avec un dispositif 7 de collecte de particules et de microorganismes présents dans l'air ambiant.

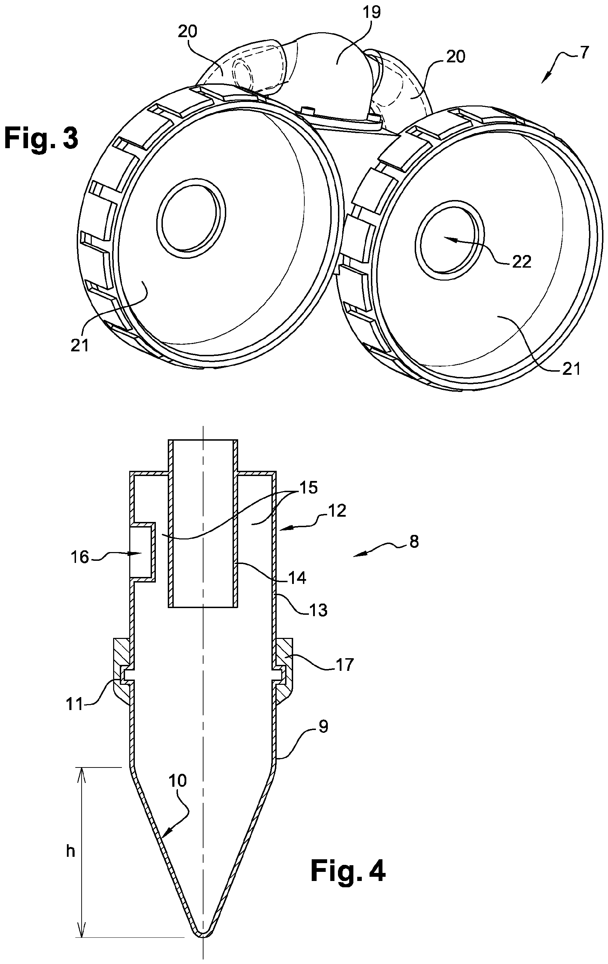

Ce dispositif 7 comporte une enceinte cyclonique 8 de centrifugation d'air de forme conique ou tronconique, dont la structure est mieux visible à la figure 4.

Cette enceinte 8 est délimitée, dans sa partie inférieure, par une première pièce 9 de forme générale tronconique ou conique et dont la surface interne 10 est recouverte d'un matériau électrostatique, par exemple du type « électret », ou d'un matériau à base de nanofibres destiné à collecter les particules ou les microorganismes. Le diamètre supérieur de la première pièce 9 est compris entre 10 et 50 mm, sa hauteur h est comprise entre 10 et 100 mm. L'extrémité supérieure de la première pièce 9 est pourvue d'un rebord annulaire 11 s'étendant radialement vers l'extérieur.

L'enceinte 8 est également délimitée, dans sa partie supérieure, par une seconde pièce 12, surmontant la première, comportant une paroi externe 13 cylindrique et un tube axial 14 délimitant avec la paroi externe un canal annulaire d'entrée d'air 15 débouchant dans l'enceinte. La paroi externe 13 comporte au moins une ouverture 16 débouchant tangentiellement dans le canal annulaire 15, de façon à former une entrée d'air dans la partie supérieure de l'enceinte 8. L'extrémité inférieure de la seconde pièce 12 est pourvue d'un filetage externe.

Les deux pièces 9, 12 sont fixées l'une à l'autre par l'intermédiaire d'une bague 17 montée par sertissage ou encliquetage sur le rebord 11 de la première pièce 9 et vissée sur le filetage de la seconde pièce 12.

Le tube axial 14 forme une sortie d'air débouchant à son extrémité inférieure dans l'enceinte et reliée à son extrémité supérieure à un raccord visible aux figures 2 et 3. Ce raccord 19 est en forme générale de T dont une branche est reliée à la sortie d'air 14 de l'enceinte 8 et dont les deux autres branches sont reliées aux deux filtres 4 par l'intermédiaire de canalisations coudées 20 et d'embouts de montage 21. Le raccord 19 est fixé, par exemple par vissage, sur la seconde pièce 12 de l'enceinte cyclonique 8.

Les embouts 21 sont cylindriques et forment des cloches emboîtables directement sur les filtres 4. Plus particulièrement, chaque embout 21 peut être réalisé en un matériau élastomère et comporte une sortie d'air 22 centrale (figure 3) située en regard de l'entrée d'air centrale 5 des filtres 4.

Lors du fonctionnement de l'appareil respiratoire A, l'air aspiré par les moyens motorisés 3 entre par l'entrée 16 de l'enceinte cyclonique 8 et tourbillonne dans cette enceinte de sorte que les particules et microorganismes présents dans l'air ambiant soient amenés par centrifugation sur le matériau électrostatique ou sur le matériau à base de nanofibres recouvrant la surface interne 10 de la pièce 9.

Contrairement à l'art antérieur précité, l'enceinte 8 fonctionne à sec, c'est-à-dire sans qu'aucun liquide ne soit contenu ou injecté dans l'enceinte.

En variante, bien que cela soit moins efficace, la collecte peut également être réalisée dans une enceinte 8 dépourvue de matériau électrostatique sur sa surface interne 10.

L'air sort ensuite de l'enceinte par la sortie 14 et traverse les canalisations 20 puis les filtres 4 afin d'être purifié avant d'atteindre le masque. Le débit d'air nécessaire à la bonne ventilation de l'utilisateur est inférieur à 100 litres par minutes, même en cas d'effort physique relativement intense. Les moyens d'alimentation motorisés 3 et l'enceinte cyclonique 8 sont dimensionnés pour autoriser un tel débit.

Après avoir terminé sa mission, l'utilisateur arrête les moyens d'alimentation motorisés 3 de l'appareil de protection respiratoire et l'enceinte 8 peut être démontée. La première pièce 9 est rincée à l'aide d'un liquide approprié afin de récupérer les particules et/ou les microorganismes collectés sur la surface interne 10 sous forme d'un échantillon liquide qui peut être analysé et/ou dosé par des techniques conventionnelles. De cette manière, les particules ou les microorganismes présents dans l'échantillon peuvent être identifiés et dénombrés.

Afin d'améliorer le décollement des particules ou des microorganismes, la première pièce 9 peut être soumise à des ultrasons, après ou lors de son rinçage.

L'invention permet de collecter des particules ou des microorganismes de façon continue et efficace, tout au long d'une mission dont la durée peut atteindre 10 heures.

Le dispositif de collecte 7, et plus particulièrement l'enceinte cyclonique 8, peuvent être conçus de façon à autoriser le fonctionnement de l'appareil de protection respiratoire 2, même en cas d'arrêt des moyens d'alimentation motorisés 3, par simple aspiration d'air par le porteur. Les pertes de charges créées par le dispositif de collecte 7 doivent donc être suffisamment faibles pour cela. Dans le cas contraire, le dispositif de collecte 7 selon l'invention peut être équipé de moyens de déconnexion rapide permettant à l'air ambiant d'être aspiré directement au travers des filtres 4, sans passer par l'enceinte cyclonique 8. Les filtres 4 sont en effet conçus pour générer des pertes de charge suffisamment faibles pour permettre le fonctionnement de l'appareil 2 en cas de panne des moyens motorisés d'alimentation 3.

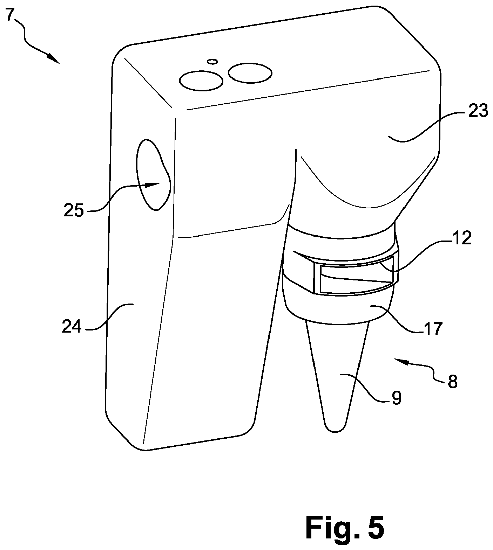

Un dispositif 7 de collecte de particules et de microorganismes selon une variante de réalisation de l'invention est représenté à la figure 5. Dans ce dispositif 7, la sortie d'air de l'enceinte 8 est raccordée à l'entrée d'air d'une turbine d'aspiration à moteur électrique, logée dans un boîtier 23 et alimentée par des batteries, elles-mêmes logées dans un boîtier 24. Les boîtiers 23 et 24 peuvent être contigus ou écartés l'un de l'autre. La sortie d'air de la turbine débouche en 25 à l'extérieur, en partie supérieure du boîtier 24.

Le dispositif 7 est destiné à être porté par une personne et à fonctionner en continu pendant une durée de plusieurs heures.

## Revendications

1. Appareil individuel motorisé de protection respiratoire (A) équipant un gilet destiné à être porté par une personne, comportant un masque, destiné à être porté par un utilisateur, et des moyens motorisés d'alimentation en air (3), par exemple à turbine, permettant d'aspirer de l'air extérieur au travers d'au moins un filtre (4) destiné à retenir les particules ou les microorganismes présents dans l'air extérieur, **caractérisé en ce qu'**il comporte un dispositif portable (7) de collecte de particules et de microorganismes présents dans l'air ambiant, monté sur le filtre (4), en amont de celui-ci dans le sens d'aspiration de l'air extérieur, le dispositif de collecte (7) comportant une enceinte cyclonique (8) de centrifugation d'air de forme conique ou tronconique, des moyens (16) d'entrée d'air extérieur dans l'enceinte (8), des moyens (14) de sortie d'air de l'enceinte (8), et des moyens de raccordement (19, 20, 21) des moyens de sortie d'air de l'enceinte à des moyens d'entrée d'air (4, 5) de l'appareil individuel motorisé de protection respiratoire (A), les moyens de raccordement de la sortie d'air de l'enceinte comprennent des moyens de branchement (21) sur au moins un filtre d'entrée (4) de l'appareil de protection respiratoire, les moyens de branchement comportent au moins un embout (21) de montage en forme de cloche, venant s'emboîter directement sur le filtre d'entrée (4) de l'appareil (A).

2. Appareil (A) selon la revendication 1, **caractérisé en ce que** la surface interne (10) de l'enceinte cyclonique (8) est constituée ou est recouverte d'un matériau électrostatique ou d'un matériau à base de nanofibres destiné à collecter les particules ou les microorganismes.

3. Appareil (A) selon la revendication 1 ou 2, **caractérisé en ce que** les moyens (16) d'entrée d'air extérieur débouchent tangentiellement dans la partie supérieure (12) de l'enceinte (8) et les 25 moyens (14) de sortie d'air débouchent axialement dans la partie supérieure (12) de l'enceinte (8).

4. Appareil (A) selon l'une des revendications 1 à 3, **caractérisé en ce que** la partie supérieure de l'enceinte (8) a un diamètre compris entre 10 et 50 mm.

5. Appareil (A) selon la revendication 3 ou 4, **caractérisé en ce que** la hauteur ou la dimension axiale de l'enceinte (8) est comprise entre 10 et 100 mm.

6. Appareil (A) selon l'une des revendications 1 à 5, **caractérisé en ce que** la sortie d'air de l'enceinte peut également être raccordée à l'entrée d'air d'une turbine d'aspiration à moteur alimenté par des batteries, l'ensemble étant destiné à être porté par une personne et à fonctionner en continu pendant une durée de plusieurs heures.

7. Appareil selon l'une des revendications 1 à 6, **caractérisé en ce que** les moyens d'alimentation en air (3), le filtre (4) et le dispositif de collecte (7) sont dimensionnés pour fournir à l'utilisateur un débit d'air continu inférieur à 100 litres par minute.

8. Appareil selon l'une des revendications 1 à 7, **caractérisé en ce qu'**il est destiné à être porté et utilisé pendant des périodes de temps continues pouvant atteindre 8 à 10 heures environ, et **en ce que** la collecte des particules et des microorganismes de l'air extérieur est faite en continu et à sec dans l'enceinte cyclonique (8) du dispositif précité (7) pendant toute la durée d'utilisation de l'appareil de protection respiratoire (2).

9. Procédé de collecte et de dosage des particules et des microorganismes présents dans l'air ambiant, **caractérisé en ce qu'**il consiste, après utilisation de l'appareil de protection respiratoire (A) selon l'une des revendications 1 à 8, à démonter l'enceinte cyclonique (8), à rincer sa surface interne (10) avec un liquide approprié afin de récupérer les particules et/ou les microorganismes collectés sur cette surface, et à analyser et/ou doser le contenu de l'échantillon ainsi produit.

10. Procédé selon la revendication 9, **caractérisé en ce qu'**il consiste à soumettre l'enceinte (8) à des ultrasons, après ou lors de son rinçage, de façon à décoller les particules et/ou les microorganismes de sa surface interne (10).

## Patentansprüche

1. Motorbetriebenes einzelnes Atemschutzgerät (A), das sich an einer Weste befindet, die dazu bestimmt ist, von einer Person getragen zu werden, welches eine Maske umfasst, die dazu bestimmt ist, von einem Benutzer getragen zu werden, und motorbetriebene Luftzufuhrmittel (3), zum Beispiel mit Turbine, die das Ansaugen der Außenluft durch mindestens einen Filter (4) ermöglichen, der dazu bestimmt ist, die in der Außenluft befindlichen Partikel oder Mikroorganismen zurück zu halten, **dadurch gekennzeichnet, dass** es eine tragbare Vorrichtung (7) zum Sammeln der in der Umgebungsluft enthaltenen Partikel und Mikroorganismen aufweist, die auf den Filter (4) vor besagter Vorrichtung in Saugrichtung der Außenluft montiert ist, wobei die Sammelvorrichtung (7) ein Zyklongehäuse (8) in konischer oder kegelstumpfförmiger Form zur Zentrifugierung von Luft umfasst, Mittel (16) der Außenluftzufuhr in das Gehäuse (8), Mittel (14) des Luftaustritts aus dem Gehäuse (8), Mittel zum Anschluss (19, 20, 21) der Luftaustrittsmittel des Gehäuses an Lufteintrittsmittel (4, 5) des motorbetriebenen einzelnen Atemschutzgeräts (A), wobei die Mittel zum Anschluss des Luftaustritts des Gehäuses Mittel zum Anschluss (21) an mindestens einen Eingangsfilter (4) des Atemschutzgeräts aufweisen, und die Anschlussmittel mindestens ein glockenförmiges Montageendstück (21) aufweisen, das direkt auf den Eingangsfilter (4) des Geräts (a) aufgeschoben wird.

2. Gerät (A) nach Anspruch 1, **dadurch gekennzeichnet, dass** die Innenfläche (10) des Zyklongehäuses (8) aus einem elektrostatischen Material oder einem Material aus Nanofasern besteht bzw. damit bedeckt ist, wobei dieses dazu bestimmt ist, die Partikel oder Mikroorganismen zu sammeln.

3. Gerät (A) nach Anspruch 1 oder 2, **dadurch gekennzeichnet, dass** die Mittel (16) für den Außenlufteintritt tangential in den oberen Bereich (12) des Gehäuses (8) führen und die 25 Mittel (14) des Luftaustritts axial in den oberen Bereich (12) des Gehäuses (8) führen.

4. Gerät (A) nach einem der Ansprüche 1 bis 3, **dadurch gekennzeichnet, dass** der obere Bereich des Gehäuses (8) einen Durchmesser von 10 bis 50 mm hat.

5. Gerät (A) nach Anspruch 3 oder 4, **dadurch gekennzeichnet, dass** die Höhe oder die axiale Abmessung des Gehäuses (8) zwischen 10 und 100 cm beträgt.

6. Gerät (A) nach einem der Ansprüche 1 bis 5, **dadurch gekennzeichnet, dass** der Luftaustritt des Gehäuses auch an den Lufteintritt einer Saugturbine mit Motor angeschlossen werden kann, welcher über Batterien gespeist wird, wobei die Einheit dazu bestimmt ist, von einer Person über mehrere Stunden hinweg getragen zu werden und kontinuierlich zu funktionieren.

7. Gerät nach einem der Ansprüche 1 bis 6, **dadurch gekennzeichnet, dass** die Luftzufuhrmittel (3), der Filter (4) und die Sammelvorrichtung (7) so bemessen sind, dass sie dem Benutzer einen kontinuierlichen Luftdurchsatz von unter 100 Litern pro Minute liefern.

8. Gerät nach einem der Ansprüche 1 bis 7, **dadurch gekennzeichnet, dass** es dazu bestimmt ist, über eine Dauer von 8 bis 10 Stunden kontinuierlich getragen zu werden, und darin, dass die Partikel und Mikroorganismen der Außenluft kontinuierlich und trocken im Zyklongehäuse (8) der vorgenannten Vorrichtung (7) für die Dauer der Benutzung des Atemschutzgerätes (2) aufgenommen werden.

9. Verfahren zum Sammeln und Dosieren von Partikeln und Mikroorganismen, die in der Umgebungsluft vorhanden sind, **dadurch gekennzeichnet, dass** es nach Benutzung des Atemschutzgeräts (A) nach einem der Ansprüche 1 bis 8 darin besteht, das Zyklongehäuse (8) zu demontieren, die Innenflüche (10) mit einer geeigneten Flüssigkeit abzuspülen, um die Partikel und/oder Mikroorganismen, die sich auf dieser Fläche angesammelt haben, zurück zu gewinnen und die Zusammensetzung der so entstandenen Probe zu analysieren und/oder zu dosieren.

10. Verfahren nach Anspruch 9, **dadurch gekennzeichnet, dass** es darin besteht, das Gehäuse (8) nach oder während des Abspülens Ultraschall auszusetzen, so dass sich die Partikel und/oder Mikroorganismen von deren Innenfläche (10) lösen.

## Claims

1. An individual motorized breathing apparatus (A) provided on a vest intended to be worn by a person, comprising a mask intended to be worn by a user, and motorized air-supplying, for instance turbine-powered, means (3) making it possible to suck the outside air through at least one filter (4) intended to hold the particles and the microorganisms present in the outside air, **characterized in that** it comprises a portable device (7) for collecting the particles and the microorganisms present in the ambient air, mounted on the filter (4), upstream thereof in the outside air suction direction, with the collecting device (7) comprising an air centrifugation cyclonic chamber (8) having a conical or a frustoconical shape, outside air inlet means (16) letting air into the chamber (8), air outlet means (14) letting air out of the chamber (8), and means (19, 20, 21) for connecting the air outlet means of the chamber to air inlet means (4, 5) of the individual motorized breathing apparatus (A), with the air outlet connection means of the chamber comprising connection means (21) on at least one inlet filter (4) of the breathing apparatus, with the connection means comprising at least one bell-shaped mounting tip (21) to be directly fitted onto the input filter (4) of the apparatus (A).

2. An apparatus (A) according to claim 1, **characterized in that** the inner surface (10) of the cyclonic chamber (8) is made of or is coated with an electrostatic material or a nanofiber-based material intended for collecting the particles or the microorganisms.

3. An apparatus (A) according to claim 1 or 2, **characterized in that** the outside air inlet means (16) tangentially open into the upper part (12) of the chamber (8) and the 25 air outlet means (14) axially open into the upper portion (12) of the chamber (8).

4. An apparatus (A) according to one of claims 1 to 3, **characterized in that** the upper part of the chamber (8) has a diameter between 10 and 50mm.

5. An apparatus (A) according to claim 3 or 4, **characterized in that** the height or the axial dimension of the chamber (8) ranges from 10 to 100mm.

6. An apparatus (A) according to one of claims 1 to 5, **characterized in that** the air outlet of the chamber can also be connected to the air inlet of a suction turbine having an engine powered by batteries, with the assembly being intended to be worn by a person and to operate continuously for a period of several hours.

7. An apparatus according to one of claims 1 to 6, **characterized in that** the air supply means (3), the filter (4) and the collecting device (7) are so dimensioned as to provide the user with a continuous flow of air of less than 100 liters per minute.

8. An apparatus according to claims 1 to 7, **characterized in that** it is intended to be worn and used for continuous periods of time up to about 8 to 10 hours, and **in that** the dry collection of particles and microorganisms from the outside air is made continuously and in the cyclonic chamber (8) of the aforesaid device (7) through the whole duration of utilization of the breathing apparatus (2).

9. A method for collecting and metering the particles and microorganisms present in the ambient air, **characterized in that** it consists, after using the breathing apparatus (A) according to one of claims 1 to 8, in disassembling the cyclonic chamber (8), in rinsing the inner surface (10) thereof with a suitable liquid in order to recover the particles and/or the microorganisms collected on this surface, and in analyzing and/or metering the content of the sample so obtained.

10. The method according to claim 9, **characterized in that** it consists in subjecting the chamber (8) to ultrasound, after or during rinsing, so as to detach the particles and/or the microorganisms from the inner surface (10) thereof.
